# EUROPEAN PATENT APPLICATION

(11) **EP 4 837 537 A1**
(43) Date of publication of application: **11.11.2026**
(21) Application number: 25803321.6
(22) Date of filing: 09.04.2025
(51) Int. Cl.: A61F 2/16

(54) **INTRAOCULAR LENS INSERTER, INTRAOCULAR LENS, AND INTRAOCULAR LENS INSERTION METHOD**

(30) Priority: 13.05.2024 JP 2024078024
(71) Applicant: Hoya Medical Singapore Pte. Ltd., Singapore 138670 (SG)
(72) Inventor: WATANABE Yoshitaka, Tokyo 160-8347 (JP)
(74) Representative: MERH-IP Matias Erny Reichl Hoffmann Patentanwälte PartG mbB
(86) International application number: PCT/JP2025/014168
(87) International publication number: WO 2025/239070

(57) **Abstract**

There is provided an intraocular lens inserter including: an inserter main body 2 having a hollow cylindrical portion capable of containing an intraocular lens; moving mechanism portions 20, 30 that move the intraocular lens within the hollow cylindrical portion; a rotatable actuator 50 attached to the inserter main body so as to be rotatable around the inserter main body 2; and a cap member 3 attached to the inserter main body 2, wherein the moving mechanism portions 20, 30 move the intraocular lens in conjunction with a rotation of the rotatable actuator 50.

## Description

### Technical Field

The present invention relates to an intraocular lens inserter, an intraocular lens, and an intraocular lens insertion method.

### Background Art

In cataract surgery, a lens that has become cloudy is extracted, and then an intraocular lens is inserted into the lens capsule in place of the lens. An intraocular lens inserter is used to insert the intraocular lens.

One invention relating to an intraocular lens cartridge has a configuration in which when the cap is detached, the nozzle is exposed and the intraocular lens moves and folds within the housing (e.g., see Patent Document 1).

### Citation List

### Patent Literature

Patent Document 1: JP 2022-545417A

### Summary of Invention

### Technical Problem

There may be cases where the user of the intraocular lens inserter (an operator such as a doctor) desires that the folding of the intraocular lens and the detachment of the cap can be performed at separate timings (e.g., with a delay between the timings). For example, after completing an operation performed prior to insertion of the intraocular lens (e.g., after removing the lens), it may be cumbersome to set up the intraocular lens inserter from scratch, and thus the intraocular lens may be held in a folded state beforehand. However, it is difficult for the intraocular lens inserter having the above-mentioned configuration to be used in such a case, and it cannot necessarily be said that the above-described intraocular lens inserter is convenient for the user.

It is also conceivable to perform the folding of the intraocular lens and the detachment of the cap as separate operations, but in such a case, the ease of operation is impaired. In the case where the lens is folded by pulling off the cap, depending on how the operation is performed by the user, there is a risk of impact with the intraocular lens, damage thereto, or the like, and thus care must be taken when performing operations, and in this respect it cannot necessarily be said that the operability is excellent.

The present invention provides technology that can achieve excellent convenience and operability when using an intraocular lens inserter.

### Solution to Problem

A first aspect of the present invention is
an intraocular lens inserter including: an inserter main body having a hollow cylindrical portion capable of containing an intraocular lens, and configured to be able to release the intraocular lens contained in the hollow cylindrical portion to outside from a lens outlet;
moving mechanism portion that moves the intraocular lens within the hollow cylindrical portion, along a cylindrical axis direction of the hollow cylindrical portion;
a rotatable actuator attached to the inserter main body so as to be rotatable in a direction around a cylindrical axis of the hollow cylindrical portion;
   and
a cap member attached to the inserter main body so as to cover at least the lens outlet,
wherein the moving mechanism portion moves the intraocular lens in conjunction with a rotation of the rotatable actuator, and the cap member can be attached to and detached from the inserter main body while the rotatable actuator is at a predetermined rotation position.

A second aspect of the present invention is the intraocular lens inserter according to the first aspect,
wherein the moving mechanism portion has an engagement piece that protrudes outward of a cylinder of the hollow cylindrical portion, and
when the engagement piece engages with an engagement groove provided in the rotatable actuator, the rotatable actuator and the moving mechanism portion are in conjunction each other.

A third aspect of the present invention is the intraocular lens inserter according to the second aspect,
wherein the moving mechanism portion includes a slider mechanism portion that moves the intraocular lens within the hollow cylindrical portion.

A fourth aspect of the present invention is the intraocular lens inserter according to the third aspect,
wherein the engagement groove in the rotatable actuator extends spirally in a cylindrical axis direction of the hollow cylindrical portion.

A fifth aspect of the present invention is the intraocular lens inserter according to the first aspect,
wherein the cap member has:
a tacking pin arranged to protrude into a cylinder of the hollow cylindrical portion in the attached state; and
a pin support portion that supports the tacking pin,
wherein when the cap member is detached from the inserter main body, the tacking pin is retracted from inside of the cylinder of the hollow cylindrical portion due to deformation of the pin support portion.

A sixth aspect of the present invention is the intraocular lens inserter according to the fifth aspect,
wherein in addition to the tacking pin and the pin support portion, the cap member has a guide portion that causes deformation of the pin support portion in response to a relative movement between the cap member and the hollow cylindrical portion in the cylindrical axis direction of the hollow cylindrical portion.

A seventh aspect of the present invention is the intraocular lens inserter according to the second aspect,
wherein the moving mechanism portion includes a pushing mechanism portion that pushes out the intraocular lens from the lens outlet.

An eighth aspect of the present invention is the intraocular lens inserter according to the seventh aspect, including
a locking mechanism portion that restricts a pushing operation for the intraocular lens by the pushing mechanism portion.

A ninth aspect of the present invention is the intraocular lens inserter according to the eighth aspect,
wherein the locking mechanism portion is configured to cancel a restriction on the pushing operation, in conjunction with a rotation of the rotatable actuator.

A tenth aspect of the present invention is an intraocular lens inserter, which is an intraocular lens inserter for use with an intraocular lens, including an optical portion and an outer rim, the intraocular lens inserter including:
an inserter main body that defines an outer shape and a lens traveling direction, the inserter main body including:
a hollow cylindrical portion;
an intraocular lens storge area;
a nozzle;
a transition area between the intraocular lens storge area and the nozzle;
a plunger that is movable distally within the inserter main body in a lens
traveling direction relative to the inserter main body, having an end face configured to engage and push the intraocular lens during its distal movement;
a lens folding device that is movable distally in the lens traveling direction
relative to the inserter main body and the plunger, and configured to fold the intraocular lens during its distal movement; and
a rotatable actuator associated with an exterior of the inserter main body and operably connected to the lens folding device, so that the lens folding device moves distally in the lens traveling direction in response to the rotation of the rotatable actuator.

An eleventh aspect of the present invention is the intraocular lens inserter according to the tenth aspect, including
a cap member detachably fixed to the inserter main body above the nozzle.

A twelfth aspect of the present invention is the intraocular lens inserter according to the eleventh aspect,
wherein the rotatable actuator is rotatable relative to the cap member.

A thirteenth aspect of the present invention is the intraocular lens inserter according to the eleventh or twelfth aspect,
wherein the cap member includes a first cap lock that is set in a locked state that prevents the cap member from being detached from the inserter main body, and an unlocked state that allows the cap member to be detached from the inserter main body, and
the lens folding device is configured to set the first cap lock to shift from the locked state to the unlocked state during a distal movement of the lens folding device.

A fourteenth aspect of the present invention is the intraocular lens inserter according to the thirteenth aspect,
wherein the inserter main body includes a latch opening, and
the first cap lock includes a latch that extends through the latch opening when the first cap lock is set in the locked state, and
the lens folding device includes a surface on which the latch moves relative to the latch opening when the lens folding device sets the first cap lock to shift from the locked state to the unlocked state.

A fifteenth aspect of the present invention is the intraocular lens inserter according to any one of the tenth to fourteenth aspects,
wherein the second cap lock is further set in a locked state that prevents the cap member from being detached from the inserter main body, and an unlocked state that allows the cap member to be detached from the inserter main body, and
the second cap lock is set to shift from the locked state to the unlocked state by rotating the rotatable actuator.

A sixteenth aspect of the present invention is the intraocular lens inserter according to the fifteenth aspect,
wherein the second cap lock includes a pin and a slot in which the pin is placed.

A seventeenth aspect of the present invention is the intraocular lens inserter according to the sixteenth aspect,
wherein the cap member defines an inner surface, the pin is on the inner surface of the cap member, and the slot is positioned on a rotatable actuator and includes a circumferentially extending portion, a distally extending portion having an end portion connected to the circumferentially extending portion and an open end, and
when the rotatable actuator is fully rotated, the pin and the distally extending portion of the slot are aligned.

An eighteenth aspect of the present invention is the intraocular lens inserter according to any one of the tenth to seventeenth aspects,
wherein the lens folding device includes a slider including an end face configured to engage with the outer rim of the intraocular lens, a hinge, and a lens holder connected to the hinge extending distally beyond the outer edge and onto an optical component of the intraocular lens.

A nineteenth aspect of the present invention is the intraocular lens inserter according to any one of the tenth to eighteenth aspects,
wherein the hollow cylindrical portion defines a circumference and a central axis, and
the rotatable actuator extends completely around a circumference of a part of the hollow cylindrical portion and is rotatable around the central axis.

A twentieth aspect of the present invention is the intraocular lens inserter according to the nineteenth aspect,
wherein the hollow cylindrical portion includes a cylindrical wall and at least one longitudinally extending slot extending through the cylindrical wall,
the rotatable actuator includes an inner surface with a helical thread, and
the lens folding device includes at least one post extending into the helical thread through at least one longitudinally extending slot.

A twenty-first aspect of the present invention is the intraocular lens inserter according to the twentieth aspect,
wherein the hollow cylindrical portion includes first and second longitudinally extending slots extending through the cylindrical wall, and
the lens folding device includes first and second posts extending into the helical thread through the first and second longitudinally extending slots, respectively.

A twenty-second aspect of the present invention is the intraocular lens inserter according to any one of the eleventh to twenty-first aspects, further including
a detachable plug to prevent the rotatable actuator from rotating.

A twenty-third aspect of the present invention is the intraocular lens inserter according to the twenty-second aspect,
wherein the cap member includes a cylindrical wall and an opening passing through the cylindrical wall configured to receive the detachable plug,
the rotatable actuator includes a recess configured to receive a part of the detachable plug, and
the cap member, the rotatable actuator, and the plug each is constituted and arranged such that the cap member is fixed to the inserter main body, and the plug prevents a rotation of the rotatable actuator when the plug is positioned within the opening of the cap member and the recess of the rotatable actuator.

A twenty-fourth aspect of the present invention is the intraocular lens inserter according to any one of the tenth to twenty-third aspects,
wherein the plunger lock is set in a locked state that prevents the plunger from moving distally and an unlocked state that allows the plunger to move distally, and
the plunger lock is set to shift from the locked state to the unlocked state by rotating the rotatable actuator.

A twenty-fifth aspect of the present invention is the intraocular lens inserter according to any one of the tenth to twenty-fourth aspects,
which is further the intraocular lens stored in a lens storge area.

A twenty-sixth aspect of the present invention is an intraocular lens insertion method that is a method for inserting an intraocular lens into an eye using an intraocular lens inserter including an inserter main body that defines an exterior and a lens traveling direction, including a hollow cylindrical portion; an intraocular lens storge area; a nozzle; a transition area between the nozzle and the intraocular lens storge area; an intraocular lens in the lens storage area; and a plunger movable distally within the inserter main body relative to the inserter main body in a lens traveling direction and having an end face configured to engage and push the intraocular lens, wherein as the plunger moves distally, the rotatable actuator is associated with the exterior of the inserter main body, the intraocular lens insertion method including the following steps:
folding the intraocular lens without distally moving the plunger, by rotating the rotatable actuator; and after the intraocular lens has been folded,
pushing the intraocular lens through the nozzle and into the eye by moving the plunger distally.

A twenty-seventh aspect of the present invention is the intraocular lens insertion method according to the twenty-sixth aspect,
wherein the intraocular lens insertion device includes a lens folding device movable distally in a lens traveling direction with relative to the inserter main body and the plunger and configured to fold the intraocular lens during a distal movement of the lens folding device, and
the rotatable actuator is operably connected to the lens folding device such that the lens folding device moves distally in the lens traveling direction in response to the rotation of the rotatable actuator.

A twenty-eighth aspect of the present invention is the intraocular lens insertion method according to the twenty-sixth or twenty-seventh aspect,
wherein the intraocular lens insertion device includes a cap member on the inserter main body that covers the nozzle, and a cap lock that is set in a locked state so that the cap member is not detached from the inserter main body, and the intraocular lens insertion method further includes:
setting the cap lock to shift to an unlocked state in which the cap lock can be detached from the inserter main body during the folding step; and
detaching the cap member before the pushing step.

A twenty-ninth aspect of the present invention is the intraocular lens insertion method according to any one of the twenty-sixth to twenty-eighth aspects,
wherein the intraocular lens insertion device includes a plunger lock set in a locked state that prevents a distal movement of the plunger,
the method further including:
   setting the plunger lock to shift to an unlocked state that allows the plunger to move distally during the folding step.

A thirtieth aspect of the present invention is the intraocular lens insertion method according to any one of the twenty-sixth to twenty-ninth aspects,
wherein the hollow cylindrical portion defines a circumference and a central axis, and
the rotatable actuator extends completely around a part of the hollow cylindrical portion and is rotatable around the central axis,
the step of rotating the rotatable actuator further including:
   rotating the rotatable actuator around the central axis.

### Advantageous Effects of Invention

According to the present invention, it is possible to achieve excellent convenience and operability when using an intraocular lens inserter.

### Brief Description of Drawings

[FIG. 1] is an external perspective view showing an example of the configuration of an intraocular lens inserter according to an embodiment of the present invention.
[FIG. 2] is an exploded perspective view showing an example of the configuration of constituent elements of the intraocular lens inserter shown in FIG. 1.
[FIG. 3] is a side cross-sectional view showing an example of the internal configuration of the intraocular lens inserter shown in FIG. 1.
[FIG. 4] is an illustrative diagram showing an example of a specific aspect when using the intraocular lens inserter shown in FIG. 1, where FIG. 4(a) is a diagram showing removal of a stopper, FIG. 4(b) is a diagram showing rotation of a rotatable actuator, and FIG. 4(c) is a diagram showing detachment of a cap.
[FIG. 5] is an illustrative diagram showing an example of an unlocking operation in the intraocular lens inserter shown in FIG. 1.
[FIG. 6] is an illustrative diagram showing an example of a tacking pin retraction operation in the intraocular lens inserter shown in FIG. 1.

### Description of Embodiments

Hereinafter, embodiments of the present invention will be described with reference to the drawings.

### (1) Example configuration of intraocular lens inserter

First, an example of the configuration of an intraocular lens inserter according to the present embodiment will be described.

### (Overall Configuration)

The intraocular lens inserter described in the present embodiment is used when inserting an intraocular lens into an eye. In the present embodiment, a one-piece type of lens, which is formed from a soft material such as silicone elastomer or soft acrylic and includes a circular optical portion that performs an optical function and a pair of support portions extending from the outer peripheral portion of the optical portion, is handled as one example of an intraocular lens. Note that since intraocular lenses are well known, further description thereof will be omitted here.

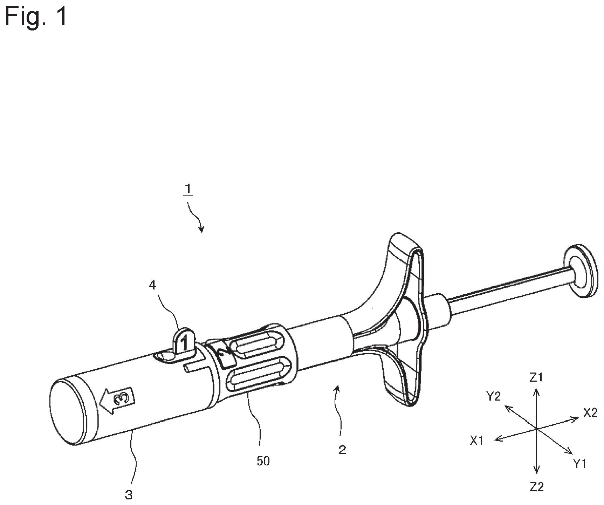

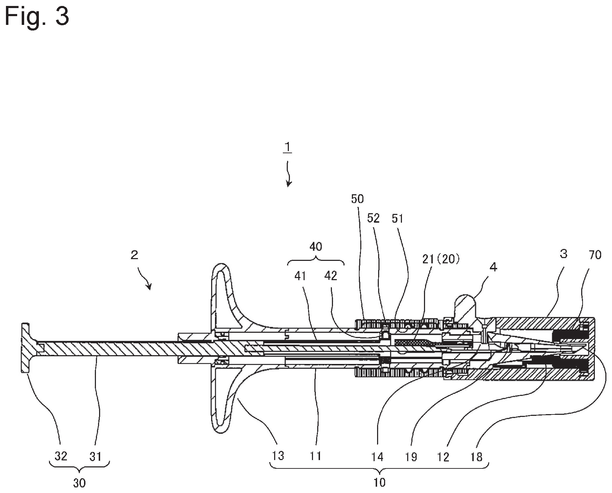

In order to insert such an intraocular lens into an eye, the intraocular lens inserter described in the present embodiment is configured as shown in FIGS. 1 to 3.

In the following description, when describing the relative positional relationships between and operating directions of portions of the intraocular lens inserter, as shown in FIG. 1, one side in an X-axis direction is defined as the X1 direction, the other side as the X2 direction, one side in an Y-axis direction is defined as the Y1 direction, the other side as the Y2 direction, one side in an Z-axis direction is defined as the Z1 direction, the other side as the Z2 direction, the X1 direction is defined as the leading end side (forward), the X2 direction is defined as the rear end side (rearward), the Y1 direction is defined as the left side (leftward), the Y2 direction is defined as the right side (rightward), the Z1 direction is defined as the upper side (upward), and the Z2 direction is defined as the lower side (downward). Among these directions, the X-axis direction (X1 direction and X2 direction) corresponds to the length direction of the intraocular lens inserter 1, the Y-axis direction (Y1 direction and Y2 direction) corresponds to the width direction of the intraocular lens inserter 1, and the Z-axis direction (Z1 direction and Z2 direction) corresponds to the height direction of the intraocular lens inserter 1.

As shown in FIGS. 1 to 3, an intraocular lens inserter 1 according to the present embodiment mainly includes an inserter main body 2 and a cap member 3. The inserter main body 2 includes at least a body portion 10, a slider mechanism portion 20, a pushing mechanism portion 30, and a locking mechanism portion 40. Furthermore, a rotatable actuator 50 is attached to the inserter main body 2. These constituent elements are preferably formed by resin molded parts.

These constituent elements will be described in detail in order below.

### (Body Portion)

The body portion 10 functions as a trunk portion of the inserter main body 2, and is formed in a hollow cylindrical shape capable of containing an intraocular lens. In other words, the inserter main body 2 has the body portion 10 that is a hollow cylindrical portion capable of containing an intraocular lens. More specifically, the body portion 10 has a base portion 11 formed in a cylindrical shape and having an opening at the rear end, a nozzle portion 12 attached to the leading end side of the base portion 11 so as to be in communication with the inside of the cylinder of the base portion 11, and a flange portion 13 formed in the vicinity of the rear end of the base portion 11. It is preferable that these portions are all made of a resin material having optical transparency so that the inside of the tube can be seen.

The base portion 11 includes, inside the tube, a lens housing portion 14 that has a placement base for an intraocular lens and is configured to house the intraocular lens on the placement base. Due to the body portion 10 having the lens housing portion 14, the intraocular lens inserter 1 of the present embodiment can function as a preload type of inserter in which the intraocular lens is placed in the inserter main body 2 in advance.

Oval slit holes 15 extending along the cylindrical axis direction of the body portion 10 (the X-axis direction) are provided in portions of the base portion 11 (e.g., the left and right wall portions). The slit holes 15 are configured to engage with slider engagement pieces 22 of the later-described slider mechanism portion 20.

Furthermore, the base portion 11 includes a guide hole 16 that extends partially around in the circumferential direction of the body portion 10, in a portion separate from the slit holes 15 (e.g., a portion closer to the flange portion 13 than the slit holes 15 are). A lock engagement piece 42 of the later-described locking mechanism portion 40 is configured to engage with the guide hole 16.

Note that the functions of the slit holes 15 and the guide hole 16 will be described in detail later. Also, in the following description, the cylindrical axis direction of the body portion 10 may be simply referred to as the "X-axis direction".

The nozzle portion 12 is used when the intraocular lens, which is housed in the lens housing portion 14 in the body portion 10, is to be inserted into an eye, and is for folding the intraocular lens into a small size and guiding the intraocular lens into the eye. Accordingly, the nozzle portion 12 has a lens outlet 18 at the leading end side (i.e., the tube end of the body portion 10) for ejecting the intraocular lens, and is configured such that the cross-sectional area of the internal space gradually narrows from the body portion 10 side toward the lens outlet 18.

A transition portion 19, which is for folding the intraocular lens that moved from the lens housing portion 14, is set at a predetermined position in the internal space of the nozzle portion 12. Specifically, the nozzle portion 12 is configured to be able to, by utilizing the gradual narrowing of the cross-sectional area of the internal space, pre-fold the intraocular lens that is moving toward the transition portion 19, which is the predetermined position.

Also, in the internal space of the nozzle portion 12, a later-described tacking pin 71 protrudes through a through hole (not shown) formed in a part of the nozzle portion 12 (e.g., the lower side wall portion) so as to correspond to the transition portion 19, which is the predetermined position. The tacking pin 71 is for enabling appropriate pre-folding of the intraocular lens (particularly folding of the front support portion of the intraocular lens).

The flange portion 13 is for supporting the fingers of the operator using the intraocular lens inserter 1 (for example, a doctor performing cataract surgery; hereinafter simply referred to as the "operator") when operating the pushing mechanism portion 30. Therefore, the flange portion 13 may have any shape or configuration as long as it can ensure the operability of the pushing mechanism portion 30, and is not limited to a specific shape, configuration, or the like.

### (Slider Mechanism Portion)

The slider mechanism portion 20 functions as one of the moving mechanism portions that moves the intraocular lens in the body portion 10 along the X-axis direction. Specifically, the moving mechanism portions include the slider mechanism portion 20 that moves the intraocular lens in the body portion 10. More specifically, the slider mechanism portion 20 is arranged inside the body portion 10 so as to be movable along the X-axis direction, and is thus configured to move the intraocular lens contained in the lens housing portion 14 to the transition portion 19, which is the predetermined position. Due to the slider mechanism portion 20 moving the intraocular lens, the intraocular lens is pre-folded in the internal space of the body portion 10.

In order to move the intraocular lens, the slider mechanism portion 20 has a lens support member 21 that applies pressing force to the intraocular lens for movement, and slider engagement pieces 22 connected to the lens support member 21. The slider engagement pieces 22 are formed so as to protrude through the slit holes 15 to the outside of the cylindrical body portion 10. Since the slider engagement pieces 22 formed in this manner are able to move freely along the X-axis direction within the range of the slit holes 15, the slider mechanism portion 20 is configured such that the lens support member 21 can move the intraocular lens by operating in conjunction with the slider engagement pieces 22.

### (Pushing Mechanism Portion)

The pushing mechanism portion 30 functions as another one of the moving mechanism portions that move the intraocular lens along the X-axis direction in the body portion 10. Specifically, the moving mechanism portions include the pushing mechanism portion 30 that pushes the intraocular lens out through the lens outlet 18. More specifically, the pushing mechanism portion 30 is configured to push the intraocular lens, which has moved to the transition portion 19, along the X-axis direction from the rear end side, thereby pushing the intraocular lens out through the lens outlet 18 of the body portion 10 to the outside (e.g., into the eye).

Known examples of the pushing mechanism portion 30 that pushes the intraocular lens out include a push type of mechanism that pushes the intraocular lens by pushing in a plunger or the like, a screw type of mechanism that pushes out the intraocular lens by rotating a rotating member or the like, and a type of mechanism that is compatible with both a push type and a screw type. Hereinafter, in the present embodiment, the case where the pushing mechanism portion 30 is a push type of mechanism will be taken as an example. However, the pushing mechanism portion 30 is not limited to being of a push type, and may be of a screw type, or may be compatible with both a push type and a screw type.

The push-type pushing mechanism portion 30 has, as movable bodies that can move along the X-axis direction in the body portion 10, at least a rod-shaped rod portion 31 extending along the X-axis direction and a plunger end 32 provided on the rear end side of the rod portion 31. The plunger end 32 and a part of the rod portion 31 are arranged to protrude from the rear end of the body portion 10, and the other part of the rod portion 31 is arranged to be contained in the body portion 10.

The leading end side of the rod portion 31 is formed in a shape that abuts against and presses the intraocular lens. The pressing force applied to the plunger end 32 is transmitted to the intraocular lens, thereby pushing the intraocular lens out of the body portion 10.

The plunger end 32 is for the operator to perform a push-in operation. For this reason, the plunger end 32 is configured with a shape that allows the operator to apply a pressing force to the rear end side thereof by hand.

In this way, the plunger end 32 and a part of the rod portion 31 protrude from the rear end of the body portion 10 so that the operator can perform the push-in operation on them. In other words, the portions protruding from the rear end of the body portion 10 (i.e., the plunger end 32 and a part of the rod portion 31) function as an operating member that allows the operator to perform the push-in operation. When the push-in operation is performed on the operating member, in conjunction therewith, the protruding portion of the rod portion 31 is pushed into the body portion 10, and the leading end of the rod portion 31 pushes the intraocular lens inside the body portion 10 so as to be ejected to the outside.

In the pushing mechanism portion 30 configured as above, a rib 33 extending along the X-axis direction is provided on a part of the rod portion 31. Note that the function of the rib 33 of the rod portion 31 will be described in detail later.

Also, in the pushing mechanism portion 30, a slider portion 34 having a shape that fits into the cylinder of the base portion 11 in the body portion 10 is provided on a part of the rod portion 31, and a configuration is possible in which the gap between the outer circumferential surface of the slider portion 34 and the inner cylindrical surface of the base portion 11 is sealed by an O-ring 35. According to this configuration, it is possible to prevent leakage after the tube is filled with a later-described viscoelastic substance (OVD). Furthermore, by utilizing the sliding resistance of the O-ring 35, it is possible to control the ejection force of the intraocular lens so as to not become excessive.

### (Locking Mechanism Portion)

The locking mechanism portion 40 is configured to restrict the push-out operation performed on the intraocular lens by the pushing mechanism portion 30, as necessary. The push-out operation is restricted by restricting the operation of the operating member (i.e., the plunger end 32 and a part of the rod portion 31). Specifically, the locking mechanism portion 40 is capable of placing the execution of the push-in operation on the operating member in a restricted state (hereinafter simply referred to as the "restricted state") and, by canceling the restriction, placing the operating member in an operable state (hereinafter simply referred to as the "operable state"), and is configured to be able to switch between the restricted state and the operable state as appropriate. As a result, for example, in the restricted state, the push-in operation cannot be performed on the operating member, and as a result, the pushing-out of the intraocular lens by the pushing mechanism portion 30 is restricted. Also, for example, in the operable state, by performing the push-in operation on the operating member, the intraocular lens can be pushed out by the pushing mechanism portion 30.

The locking mechanism portion 40 has a locking member 41 disposed in the body portion 10 in order to switch between the restricted state and the operable state.

The locking member 41 is contained in the base portion 11 of the body portion 10 so as to be positioned in the region where the guide hole 16 is formed in the base portion 11, and is configured to be rotatable around the cylindrical axis of the body portion 10 in the tube of the base portion 11. Furthermore, the locking member 41 includes a lock engagement piece 42 formed so as to protrude through the guide hole 16 to the outside the tube of the base portion 11. The lock engagement piece 42 is movable within the range of the guide hole 16, and thus a rotational force is applied to the locking member 41 in the tube of the base portion 11, and the rotation position is controlled.

Also, the locking member 41 is configured such that rod portion 31 of the pushing mechanism portion 30 passes through a portion thereof near the center of rotation, and the position of the locking member 41 relative to the rod portion 31 in the X-axis direction is displaceable. Furthermore, a guide groove 43 that fits with the rib 33 of the rod portion 31 and extends along the X-axis direction is formed in the locking member 41.

By using the locking member 41 having such a configuration, the locking mechanism portion 40 is configured to switch between the restricted state and the operable state according to the rotation position of the locking member 41. Specifically, when the locking member 41 is at a rotation position according to which the rib 33 of the rod portion 31 and the guide groove 43 of the locking member 41 are engaged with each other, the rod portion 31 is displaceable in the X-axis direction, and the operating members including the rod portion 31 enter the operable state. On the other hand, when the locking member 41 is at other rotation positions, the rib of the rod portion 31 and the guide groove of the locking member 41 are not engaged with each other, and thus the displacement of the rod portion 31 in the X-axis direction is restricted by such interference, and the operating members including the rod portion 31 enter the restricted state.

The locking mechanism portion 40 that restricts execution of the push-in operation on the operating member is constituted by the locking member 41, the lock engagement piece 42 provided on the locking member 41, the rib 33 of the rod portion 31, and the guide groove 43 of the locking member 41, which are described above. Note that the guide hole 16 provided in the body portion 10 may be included as a constituent element of the locking mechanism portion 40.

### (Inserter Main Body)

The inserter main body 2, which includes the body portion 10, the slider mechanism portion 20, the pushing mechanism portion 30, and the locking mechanism portion 40 described above, is configured such that after the locking mechanism portion 40 switches from the restricted state to the operable state, first, the intraocular lens in the lens housing portion 14 is moved to the transition portion 19 by the slider mechanism portion 20, which is the predetermined position, and is thus reliably folded into a predetermined shape, and then the intraocular lens is further pushed out by the pushing mechanism portion 30 so as to be folded even smaller, and the intraocular lens in that state can be ejected from the lens outlet 18 and inserted into the eye. In this manner, the inserter main body 2 is configured to be able to contain the intraocular lens therein, and is also configured to be able to eject the contained intraocular lens to the outside through the lens outlet 18. The inserter main body 2 is configured to be able to fold the intraocular lens into a predetermined shape while moving the intraocular lens in the lens traveling direction (X-axis direction) in two stages, first by the slider mechanism portion 20 and then by the pushing mechanism portion 30, and then eject the folded intraocular lens to the outside.

Also, with respect to the body portion 10, the slider mechanism portion 20, and the pushing mechanism portion 30 that constitute the inserter main body 2, aspects thereof other than the points described here may be configured using publicly known technology (for example, see Japanese Patent No. 5413918 and Japanese Patent No. 6276619).

### (Rotatable Actuator)

The rotatable actuator 50 is attached to the inserter main body 2 so as to be rotatable in a direction around the X axis of the body portion 10 (i.e., in the circumferential direction). The rotatable actuator 50 can be rotated by the operator by hand. Note that the rotatable actuator 50 is rotatable around the X axis relative to the body portion 10, but does not move in the X-axis direction. The specific configuration (mechanism) for making the rotatable actuator 50 rotatable may be realized using known technology, and is not limited to a specific aspect.

Also, the rotatable actuator 50 is formed in a cylindrical shape for attachment to the inserter main body 2. A slider engagement groove 51 and a lock engagement groove 52 are provided inside the tube.

The slider engagement groove 51 is a recessed groove formed in the inner cylindrical surface of the rotatable actuator 50, and is configured to engage with the slider engagement pieces 22 of the slider mechanism portion 20 (in particular, the portion that protrudes outside the tube of the body portion 10). More specifically, the slider engagement groove 51 is formed to extend spirally in the X-axis direction, and is formed with a groove width and a groove depth that allow the slider engagement pieces 22 to move along the groove when the slider engagement pieces 22 are fitted into the groove. When the slider engagement pieces 22 are fitted into the slider engagement groove 51, the rotatable actuator 50 and the slider mechanism portion 20 are engaged with each other. As a result, as will be described in detail later, the slider mechanism portion 20 moves the intraocular lens in conjunction with rotation of the rotatable actuator 50.

The lock engagement groove 52, similarly to the slider engagement groove 51, is a recessed groove formed in the inner cylindrical surface of the cap member 3. However, unlike the slider engagement groove 51, the lock engagement groove 52 is configured to engage with the lock engagement piece 42 of the locking mechanism portion 40 (particularly, the portion that protrudes outside the tube of the body portion 10). More specifically, the lock engagement groove 52 is formed to extend partially around the X-axis direction of the body portion 10 (i.e., in the circumferential direction), and is formed with a groove width and a groove depth that allow the lock engagement piece 42 to move along the groove when the lock engagement piece 42 is fitted into the groove. When the lock engagement piece 42 is fitted into the lock engagement groove 52, the rotatable actuator 50 and the locking member 41 of the locking mechanism portion 40 are engaged with each other. As a result, as will be described in detail later, the lock engagement piece 42 moves in the lock engagement groove 52 in conjunction with rotation of the rotatable actuator 50, and the locking member 41 of the locking mechanism portion 40 rotates depending on the movement position, thereby switching the locking mechanism portion 40 between the restricted state and the operable state.

Furthermore, the rotatable actuator 50 has a fitting portion 53, which fits with the opening of the later-described cap member 3, on the leading end side (X1 side) of the body portion 10. The fitting portion 53 is provided with a cap engagement groove 54.

The cap engagement groove 54 is a recessed groove formed in the outer circumferential surface of the fitting portion 53, and is configured to engage with a knob engagement piece (not shown) of the later-described cap member 3. More specifically, the cap engagement groove 54 includes a rotation directional groove 55 extending partially around the outer circumferential surface of the fitting portion 53 in the circumferential direction, and an insertion/removal directional groove 56 extending linearly along the X-axis direction from one end of the rotation directional groove 55 to the edge of the rotatable actuator 50. The rotation directional groove 55 and the insertion/removal directional groove 56 are formed to have a groove width and a groove depth that allow the knob engagement piece to move along the groove when the knob engagement piece is fitted in the groove. When the knob engagement piece of the cap member 3 engages with the rotation directional groove 55 and the insertion/removal directional groove 56 that constitute the cap engagement groove 54, the cap member 3 and the rotatable actuator 50 in the attached state are engaged with each other. As a result, as will be described in detail later, when the rotatable actuator 50 is at a predetermined rotation position, the cap member 3 can be attached to and detached from the inserter main body 2, and the cap member 3 can be removed from the body portion 10 along the cylindrical axis direction of the body portion 10.

### (Cap Member)

The cap member 3 is attached to the body portion 10 of the inserter main body 2 so as to cover the side corresponding to the lens outlet 18 in the inserter main body 2. More specifically, the cap member 3 is formed in a cylindrical shape having an opening at least at one end, and is configured to fit with the fitting portion 53 of the rotatable actuator 50 attached to the body portion 10 through the opening on the one end side. For example, if the outer shape of the fitting portion 53 of the body portion 10 and the rotatable actuator 50 is circular in cross section, the cap member 3 is formed in a cylindrical shape that fits with the fitting portion 53. The cap member 3 has a fitting depth relative to the fitting portion 53 (i.e., the tube length of the fitting portion 53 that is covered) set so as to be able to reach and cover at least the formation position of the cap engagement groove 54 in the fitting portion 53. Note that the cap member 3 need only have an opening at least at one end, the other end may be open or may be sealed. In the following, an example is described in which the other end of the cap member 3 is sealed.

The cap member 3 has one end open and a sealed other end so as to be able to seal at least the lens outlet 18 of the inserter main body 2 when attached to the body portion 10 (hereinafter sometimes simply referred to as the "attached state"). As a result, when the cap member 3 is in the attached state, the lens outlet 18 of the inserter main body 2 is protected from external forces even when the inserter main body 2 is not stored in a case or the like. Covering the lens outlet 18 with the cap member 3 is also very useful in preventing the intrusion of foreign matter into the body portion 10 of the inserter main body 2.

The cap member 3 is configured such that, when attached to the body portion 10, the operator can remove the cap member 3 from the body portion 10 by hand. In other words, the cap member 3 is configured so as to be able to be attached to and detached from the body portion 10. However, the cap member 3 can be attached to and detached from the inserter main body 2 only when the rotatable actuator 50 is at the predetermined rotation position, as will be described in detail later.

The cap member 3 is also provided with a knob engagement piece and a tacking assist portion 70.

The knob engagement piece is formed on the inner cylindrical surface of the cap member 3 so as to protrude toward the cylindrical axis center of the cap member 3. The knob engagement piece formed in this manner is able to move freely in the cap engagement groove 54 in the fitting portion 53 of the rotatable actuator 50, and thus the cap member 3 can be attached and detached when the rotatable actuator 50 is at the predetermined rotation position, but attachment and detachment to and from the inserter main body 2 is restricted when the rotatable actuator 50 is at any rotation position other than the predetermined rotation position.

The tacking assist portion 70 utilizes a tacking pin 71 to assist in pre-folding of the intraocular lens so that the pre-folding of the intraocular lens can be appropriately performed by the slider mechanism portion 20. For this reason, the tacking assist portion 70 includes the tacking pin 71 provided for the cap member 3, a pin support portion 72, and a guide portion 73.

The tacking pin 71 is disposed so as to protrude into the cylindrical body portion 10 through a through hole (not shown) of the nozzle portion 12 when the cap member 3 is in the attached state. Due to the tacking pin 71 protruding into the tube of the body portion 10, when the intraocular lens in the body portion 10 is moved by the slider mechanism portion 20, the posture of a part of the intraocular lens (e.g., the support portion on the front side) is controlled by abutment with the tacking pin 71, thereby ensuring appropriate pre-folding.

The pin support portion 72 is shaped as a rod that supports the tacking pin 71, supports the tacking pin 71 with the leading end portion of the rod, and is configured such that the rod-shaped portion can undergo elastic deformation. As a result, the tacking pin 71 supported by the pin support portion 72 is arranged to protrude into the tube of the body portion 10 when the cap member 3 is in the attached state, but when the cap member 3 is removed from the body portion 10, the tacking pin 71 retracts from the tube of the body portion 10 due to elastic deformation of the pin support portion 72.

The guide portion 73 is configured by an inclined surface provided on the leading end side of the rod-shaped pin support portion 72, and, when the cap member 3 is removed from the body portion 10, causes elastic deformation of the pin support portion 72 according to relative movement between the cap member 3 and the body portion 10 in the X-axis direction. In other words, the guide portion 73 guides the tacking pin 71 to retract by using an inclined surface that causes deformation of the pin support portion 72.

The tacking assist portion 70, which is constituted by the tacking pin 71, the pin support portion 72, and the guide portion 73, is provided as a part of the cap member 3. However, the tacking assist portion 70 may be formed as a separate piece from the main body portion that constitutes the outer shape of the cap member 3.

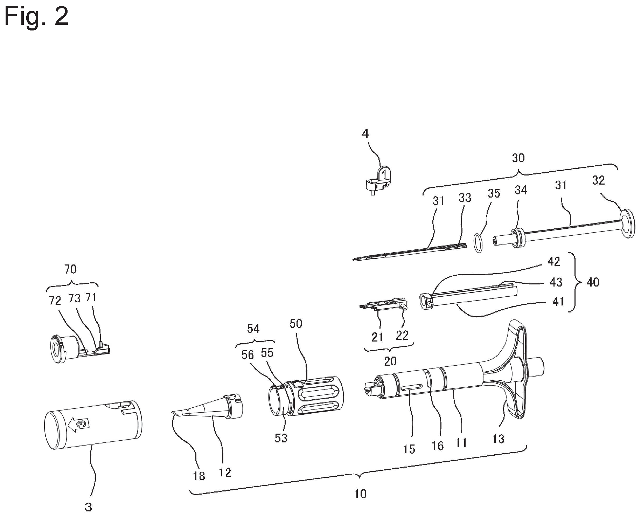

It is preferable that in addition to the above-mentioned configuration, a stopper member 4 is provided for the cap member 3 having the above-mentioned configuration, as shown in FIGS. 1 and 2.

The stopper member 4 is formed in a shape that fits into dedicated through holes 4a and 4b provided in the cap member 3 in the attached state and the body portion 10 of the inserter main body 2 to which the cap member 3 is attached. The stopper member 4 is configured so as to be able to be removed from the fitted state in the through holes 4a and 4b by being pulled out with a hand.

By providing the stopper member 4 having such a configuration, the following functions are realized when the cap member 3 is in the attached state.

For example, when fitted into the through holes 4a and 4b, the stopper member 4 protrudes into the internal space of the body portion 10, thereby functioning to restrict positional shifting of the intraocular lens (unintended movement of the intraocular lens) in the lens housing portion 14 in the body portion 10.

Note that the stopper member 4 is not limited to a specific shape, configuration or the like as long as it is configured to achieve the above-mentioned functions.

The stopper member 4 may be provided with a through hole (not shown) for communication between the inside and outside of the body portion 10 when the stopper member 4 is fitted into the through holes 4a and 4b. In the case where such a through hole is provided, it becomes possible to inject a viscoelastic substance (OVD) such as sodium hyaluronate into the internal space of the body portion 10 through the through hole. In this case, it is preferable that the through hole is formed in a tapered shape so that the needle for injecting the OVD can be easily guided and the injection operation can be facilitated. However, the OVD injection may be performed by a method other than using the through hole of the stopper member 4.

### (2) Usage of intraocular lens inserter

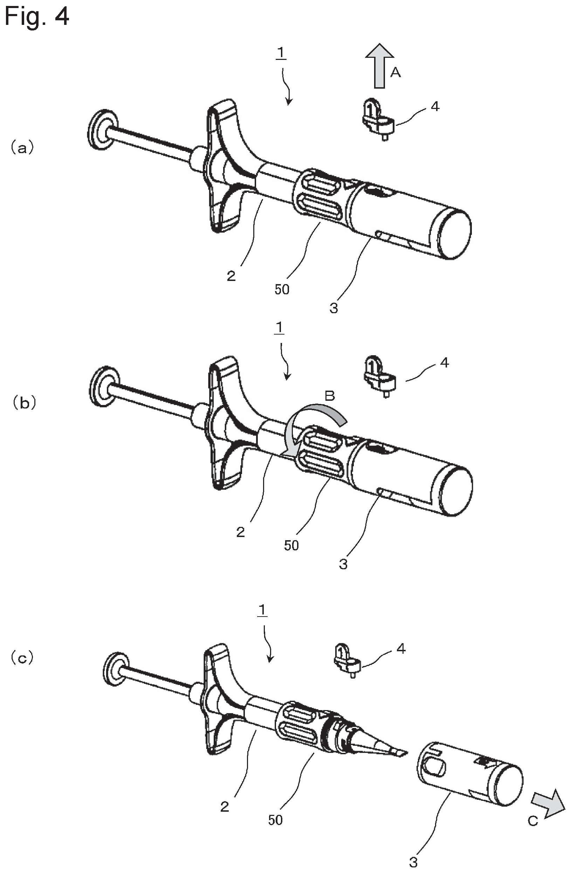

Next, a specific example of usage of the intraocular lens inserter 1 having the above-mentioned configuration will be described with reference to FIGS. 4 to 6.

When the intraocular lens inserter 1 is to be used, the intraocular lens inserter 1 is supplied housed (sealed) in a sterile bag. Hereinafter, the state in which the intraocular lens inserter 1 is housed in a sterilized bag, or the state in which the intraocular lens inserter 1 has been simply taken out of the sterilized bag without any operations being performed thereon, will be referred to as the initial state before use. Note that in the initial state, it is assumed that an intraocular lens is contained in the inserter main body 2 of the intraocular lens inserter 1. However, there is not limitation to this, and when the inserter main body 2 does not yet contain an intraocular lens, an intraocular lens may be placed in the inserter main body 2 in conjunction with the OVD injection described below or before the OVD injection.

When the intraocular lens inserter 1 is in the initial state, the cap member 3 has been attached to the body portion 10 of the inserter main body 2, and the stopper member 4 has been fitted. Therefore, in the initial state, movement of the relative positions of the inserter main body 2 and the cap member 3 is restricted by the stopper member 4, and therefore the cap member 3 cannot be removed from the inserter main body 2.

Also, when the intraocular lens inserter 1 is in the initial state, the lens push-out operation is restricted by the locking mechanism portion 40. Specifically, the rotation position of the locking member 41 in the base portion 11 constituting the locking mechanism portion 40 is set such that the operating member (i.e., the plunger end 32 and a part of the rod portion 31) is in the restricted state. This rotation position is a position where the rib 33 of the rod portion 31 and the guide groove 43 of the locking member 41 interfere with each other and do not fit together. More specifically, the lock engagement piece 42 provided with the locking member 41 is positioned in the guide hole 16 of the base portion 11 and is at a position toward one end side of the guide hole 16. When the locking member 41 and the lock engagement piece 42 are arranged in such a rotation position, the locking mechanism portion 40 places the operating member in a restricted state. Therefore, even if the cap member 3 does not cover the entirety of the inserter main body 2, and the operating member protrudes from the rear end of the body portion 10, execution of the push-in operation on the operating member is restricted. In other words, there is no risk of mis-operation of the operating member, such as being pushed in by mistake.

According to the above-described points, in the initial state, if the cap member 3 is in the attached state, a case for housing the entirety of intraocular lens inserter 1 is not necessary. In other words, the cap member 3 can be used as a substitute for such a case. Therefore, it is possible to simplify the configuration of the intraocular lens inserter 1, which is preferable in terms of reducing product cost through the reduction in resin material that accompanies simplification, and is also preferable in terms of environmental issues such as waste plastic.

When using the intraocular lens inserter 1, that is to say when using the intraocular lens inserter 1 to insert an intraocular lens into an eye, first, an OVD is injected into the internal space of the body portion 10 that houses the intraocular lens by, for example, using the through hole of the stopper member 4 to fill the internal space. This is one of the preparation steps required for using the intraocular lens inserter 1.

After filling of the OVD, removal of the stopper member 4 is performed by pulling out the stopper member 4 by hand as shown in FIG. 4(a) (see arrow A in the figure).

As a result, the stopper member 4 no longer restricts positional shifting of the intraocular lens in the body portion 10. Therefore, the intraocular lens in the body portion 10 can be released to the outside by the push-out operation performed by the pushing mechanism portion 30. Along with the injection of the OVD, this is another one of the preparation steps required for use of the intraocular lens inserter 1.

Note that even when the stopper member 4 is removed, the cap member 3 cannot be immediately removed from the inserter main body 2 by being pulled out linearly along the X-axis direction. This is because movement of the cap member 3 in the X-axis direction is restricted by engagement between the knob engagement piece of the cap member 3 and the cap engagement groove 54 of the fitting portion 53 of the rotatable actuator 50.

After the stopper member 4 is removed, the rotatable actuator 50 is then rotated by hand in one direction (e.g., counterclockwise as viewed from the leading end side) as shown in FIG. 4(b) (see arrow B in the figure). Note that during rotation, the rotatable actuator 50 does not move in the X-axis direction. In other words, rotation of the body portion 10 around the cylindrical axis and movement in the X-axis direction (the cylindrical axis direction of the body portion 10) represent mutually different operations, and are not performed simultaneously.

At this time, the slider engagement pieces 22 of the slider mechanism portion 20 protrude from the slit holes 15 of the body portion 10 of the inserter main body 2 to the outside of the cylinder of the body portion 10 through the slit holes 15. The protruding portions of the slider engagement pieces 22 are engaged with the slider engagement groove 51 in the rotatable actuator 50 so as to be positioned in the slider engagement groove 51. Therefore, the slider engagement pieces 22 can move within the range of the openings of the slit holes 15, and the slider engagement groove 51 that engages with the slider engagement pieces 22 extends spirally, and thus when the rotatable actuator 50 rotates, the engagement positions of the slider engagement pieces 22 with the slider engagement groove 51 are guided by the slit holes 15 and gradually move toward the leading end side.

As a result, inside the body portion 10, the slider mechanism portion 20 moves the intraocular lens in conjunction with rotation of the rotatable actuator 50. Rotation of the rotatable actuator 50 is performed until the movement of the intraocular lens from the lens housing portion 14 by the slider mechanism portion 20 reaches the transition portion 19. In other words, the shape and formation positions (particularly the groove end positions) of the slit holes 15 in the body portion 10 and the slider engagement groove 51 in the rotatable actuator 50 are set such that the intraocular lens reaches the transition portion 19 in conjunction with rotation of the rotatable actuator 50. In this way, when the slider mechanism portion 20 moves the intraocular lens in conjunction with rotation of the rotatable actuator 50, the movement speed of the intraocular lens depends on the number of rotations of the rotatable actuator 50. This means that the movement speed of movement of the intraocular lens by the slider mechanism portion 20 can be adjusted by setting the number of rotations of the rotatable actuator 50. Similarly to the setting of the number of rotations, the movement speed of the intraocular lens can also be adjusted by setting the slider engagement groove 51 (e.g., by setting the helical pitch).

When the intraocular lens moves to the transition portion 19, the cap member 3 is in the attached state, and the tacking pin 71 of the tacking assist portion 70 provided on the cap member 3 protrudes into the tube of the body portion 10. Moreover, even when the rotatable actuator 50 rotates, the position of protrusion of the tacking pin 71 into the body portion 10 does not change. Therefore, when the intraocular lens reaches the transition portion 19, the posture of the intraocular lens is controlled by abutment between the intraocular lens and the tacking pin 71. In other words, the tacking pin 71 allows the intraocular lens to be appropriately pre-folded.

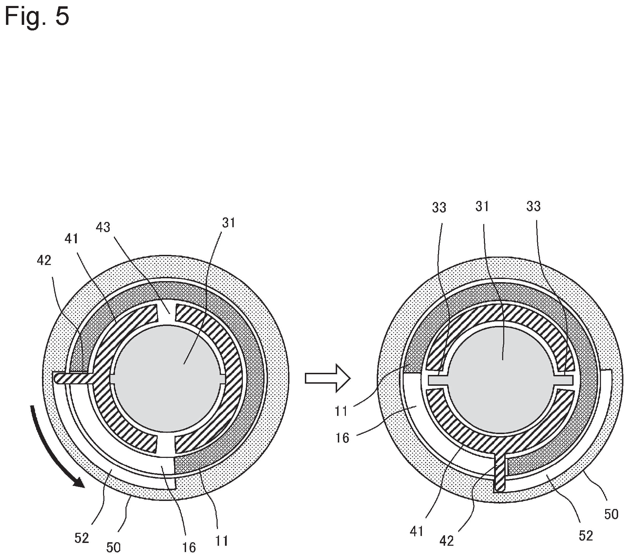

Furthermore, in the initial state before the rotatable actuator 50 rotates, execution of the lens push-out operation is restricted by the locking mechanism portion 40. Specifically, the rotation position of the locking member 41 in the base portion 11 has been set such that the operating member is in the restricted state, and the lock engagement piece 42 is positioned toward one end side in the guide hole 16. At this time, the leading end side of the lock engagement piece 42 protrudes outside the tube of the base portion 11 through the guide hole 16, as shown in FIG. 5. However, even when the lock engagement piece 42 protrudes outside the tube of the base portion 11, the protruding portion of the lock engagement piece 42 is engaged with the lock engagement groove 52 so as to be positioned in the groove of the lock engagement groove 52 in the rotatable actuator 50. Therefore, even if the lock engagement piece 42 protrudes outside the tube of the base portion 11, rotation of the rotatable actuator 50 from the initial state is not hindered by the presence of the lock engagement groove 52.

However, as the rotatable actuator 50 continues to rotate, one end side of the lock engagement groove 52 reaches the position of the lock engagement piece 42, and further rotation of the rotatable actuator 50 causes the one end of the lock engagement groove 52 to apply a pressing force to the lock engagement piece 42. As a result, the lock engagement piece 42 moves while being guided by the guide hole 16. As the lock engagement piece 42 moves, the locking member 41 of the locking mechanism portion 40 rotates in the base portion 11. In other words, in conjunction with rotation of the rotatable actuator 50, the locking member 41 of the locking mechanism portion 40 also rotates in the same direction as the rotatable actuator 50.

As shown in FIG. 5, the locking member 41 rotates until movement of the guide groove 43 formed in the locking member 41 reaches the formation position of the rib 33 in the rod portion 31 passing through the locking member 41. When the guide groove 43 reaches the rib 33, they are positioned so as to be able to fit together. As a result, the locking mechanism portion 40 switches the state of the operating member from the restricted state to the operable state.

It is preferable that the state switching performed by the locking mechanism portion 40 (i.e., the arrival of the guide groove 43 at the rib 33 as the locking member 41 rotates) coincides with the arrival of the intraocular lens at the transition portion 19. In other words, the shape and formation position (particularly the groove end position) of the guide hole 16 in the body portion 10 and the lock engagement groove 52 in the rotatable actuator 50 are set such that, in conjunction with rotation of the rotatable actuator 50, and coinciding with the intraocular lens reaching the transition portion 19, the guide groove 43 reaches the rib 33 as the locking member 41 rotates, thereby switching the locking mechanism portion 40 from the restricted state to the operable state.

When the locking mechanism portion 40 switches from the restricted state to the operable state, the restriction imposed by the locking mechanism portion 40 is cancelled, and the plunger end 32 and the rod portion 31 that constitute the pushing mechanism portion 30 become able to move along the X-axis direction.

Furthermore, as the rotatable actuator 50 rotates, the knob engagement piece of the cap member 3 moves in the rotation directional groove 55 of the fitting portion 53 of the rotatable actuator 50, and reaches the formation position of the insertion/removal directional groove 56. As a result, the knob engagement piece of the cap member 3 is able to move in the X-axis direction in the insertion/removal directional groove 56, and thus the movement restriction imposed by engagement with the cap engagement groove 54 is cancelled, making it possible to remove the cap member 3 from the body portion 10.

In other words, when the rotatable actuator 50 rotates to a position (hereinafter referred to as the "predetermined rotation position") where the guide groove 43 of the locking member 41 reaches the rib 33 of the rod portion 31 and the knob engagement piece of the cap member 3 reaches the insertion/removal directional groove 56 of the rotatable actuator 50, the cap member 3 becomes detachable from the body portion 10 at the predetermined rotation position and can be removed from the body portion 10.

After the intraocular lens reaches the transition portion 19, the restriction performed by the locking mechanism portion 40 is cancelled, and the cap member 3 can be removed, as shown in FIG. 4 (c), the attached cap member 3 is then pulled out by hand toward the leading end side to remove the cap member 3 from the inserter main body 2 (see arrow C in the figure).

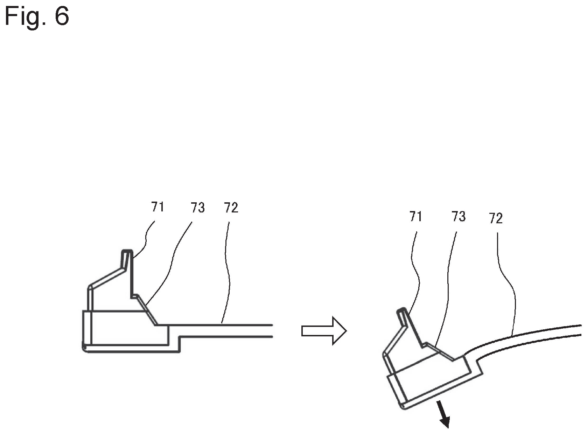

When the cap member 3 is removed, the relative positions of the cap member 3 and the inserter main body 2 move. Due to this movement of the relative position, the pin support portion 72 undergoes elastic deformation in the tacking assist portion 70 provided for the cap member 3, as shown in FIG. 6. Specifically, due to movement of the relative positions, the guide portion 73 comes into contact with the side wall surface of a through hole (not shown) provided corresponding to the tacking pin 71, and as the movement continues, the guide portion 73 is pressed along the inclined surface, thus causing the pin support portion 72 to undergo elastic deformation in the direction in which the tacking pin 71 retracts from the interior of the body portion 10. Therefore, even if the tacking pin 71 protrudes into the tube of the body portion 10, when the cap member 3 is removed, the tacking pin 71 retracts from the tube of the body portion 10 due to elastic deformation of the pin support portion 72, and thus the tacking pin 71 does not interfere with the removal of the cap member 3.

As described above, in the present embodiment, the operator performs two operations with different directions of operation, namely, rotating the rotatable actuator 50 and removing the cap member 3, on the intraocular lens inserter 1 in the initial state. These operations may be performed consecutively as a series of operations (i.e., as one continuous step), or may each be performed at an independent timing (e.g., with a delay between the timings). In either case, by performing these operations, it becomes possible to operate the slider mechanism portion 20 in conjunction with operation of the rotatable actuator 50.

Furthermore, in the present embodiment, when the intraocular lens inserter 1 is in the initial state, the operator performs two operations with different directions of operation, namely rotating the rotatable actuator 50 and removing the cap member 3, thereby making it possible to, in conjunction with the operation of the rotatable actuator 50, cancel the restriction imposed by the locking mechanism portion 40, and switch the state of the operating member from the restricted state to the operable state.

As a result, after the cap member 3 is removed, the push-in operation can be performed on the operating member. When the push-in operation is performed on the operating member, the pushing mechanism portion 30 pushes out the intraocular lens, and the intraocular lens is released from the lens outlet 18.

At this time, since the slider mechanism portion 20 operates in conjunction with the operation of the rotatable actuator 50, the intraocular lens pushed out by the pushing mechanism portion 30 has been pre-folded by the slider mechanism portion 20. Therefore, by simply performing the push-in operation on the operating member after removing the cap member 3, the intraocular lens can be released from the lens outlet 18 in a small folded state, thereby preventing folding problems when the intraocular lens is released. In other words, due to the rotatable actuator 50 and the slider mechanism portion 20 operating in conjunction with each other, the intraocular lens can be smoothly released from the lens outlet 18 simply by performing the push-in operation on the operating member.

Also, the rotatable actuator 50 operates in conjunction with the switching of the state of the operating members in addition to the slider mechanism portion 20. Specifically, when the operating member is in the operable state, the rib 33 of the rod portion 31 is guided by the guide groove 43 of the locking member 41, thereby enabling the rod portion 31 to move along the X-axis direction and push out the intraocular lens. At this time, the rod portion 31 cannot rotate relative to the body portion 10 due to the presence of the rib 33, and is in constant contact with the intraocular lens in a constant position. Therefore, even when pushing out the intraocular lens by pressing with the leading end of the rod portion 31, it is possible to stably perform the push-out operation in a constant posture, which also enables the intraocular lens to be smoothly released from the lens outlet 18.

### (3) Effects of present embodiment

According to the present embodiment, one or more of the following effects can be obtained.
(a) In the intraocular lens inserter 1 according to the present embodiment, the slider mechanism portion 20 moves the intraocular lens in conjunction with rotation of the rotatable actuator 50. However, the intraocular lens does not move in conjunction with attachment/detachment (i.e., movement in the X-axis direction) of the cap member 3. Therefore, the user of the intraocular lens inserter 1 (an operator such as a doctor) can rotate the rotatable actuator 50 (i.e., pre-fold the intraocular lens) and remove the cap member 3 as two operations with different directions of operation. These operations may be performed consecutively as a series of operations (i.e., as one continuous step), or may each be performed at an independent timing (e.g., with a delay between the timings). For example, after completing an operation performed prior to insertion of the intraocular lens (e.g., after removing the lens), it may be cumbersome to set up the intraocular lens inserter 1 from scratch, and thus it is possible to accommodate cases where the intraocular lens is held in a folded state beforehand. In other words, the intraocular lens inserter 1 is very excellent in terms of user convenience.

Also, in the intraocular lens inserter 1 of the present embodiment, the rotatable actuator 50 is arranged near the cap member 3, thus making it possible to suppress impairment of the user operability of the intraocular lens inserter 1. Furthermore, unlike the case where, for example, lens folding is performed by pulling off the cap, there is no risk of impact with intraocular lens, damage thereto, or the like caused by user operation, and the user does not need to take any special precautions when operating the device.

As described above, the intraocular lens inserter 1 according to the present embodiment can achieve excellent convenience and operability.

(b) In the intraocular lens inserter 1 according to the present embodiment, the cap member 3 is attached to the body portion 10 of the inserter main body 2 so as to cover the lens outlet 18 side. Then, by removing the cap member 3 from the body portion 10, it becomes possible to use the intraocular lens inserter 1 (i.e., to release the intraocular lens from the lens outlet 18). Therefore, compared to the case where it is necessary to remove the intraocular lens inserter 1 from a case that contains the entirety of device, it is only necessary to remove the cap member 3, which does not cause inconvenience to the operator using the intraocular lens inserter 1 (e.g., a doctor performing cataract surgery), and provides excellent operability (convenience) for the operator.

Furthermore, by covering the lens outlet 18 side with the cap member 3, it is possible to ensure a minimum required level of protective function while simplifying the configuration compared to when the lens is housed in a case, and this simplification leads to a reduction in the amount of resin material used, which is preferable in terms of reducing product cost and is also preferable in terms of environmental issues such as waste plastic.

In other words, according to the present embodiment, the intraocular lens inserter 1 can achieve excellent convenience with a simplified configuration.

(c) The intraocular lens inserter 1 of the present embodiment is configured such that the rotatable actuator 50 is rotatable around the cylindrical axis of the body portion 10 and the cap member 3 can be attached to and detached from the body portion 10 at a predetermined rotation position. In other words, even if the cap member 3 is pulled in the X-axis direction while in the attached state, the cap member 3 cannot be removed from the body portion 10, but if the rotatable actuator 50 is rotated to the predetermined rotation position, the cap member 3 can be removed from the body portion 10.

Therefore, according to the present embodiment, in the case where the inserter main body 2 is partially protected by the cap member 3, it is possible to achieve excellent operability (convenience) for the operator using the intraocular lens inserter 1 while ensuring necessary and sufficient protective function.

(d) The intraocular lens inserter 1 of the present embodiment is configured such that the rotatable actuator 50 and the slider mechanism portion 20 of the inserter main body 2 operate in conjunction with each other, and the slider mechanism portion 20 moves the intraocular lens in response to rotation of the rotatable actuator 50. Therefore, when the rotatable actuator 50 is rotated to remove the cap member 3, the intraocular lens in the body portion 10 moves accordingly to the transition portion 19, which is the predetermined position. In other words, when using the intraocular lens inserter 1, by performing the series of operations including rotating the rotatable actuator 50 and removing the cap member 3 as one continuous step, it is possible to insert an intraocular lens into the eye using the inserter main body 2. Therefore, for the operator using the intraocular lens inserter 1, operability (convenience) is even more excellent than with an intraocular lens inserter housed in a case, and the operator does not feel any inconvenience during usage. Furthermore, since the slider mechanism portion 20 is operated by rotating the rotatable actuator 50, unlike when, for example, the operator directly operates the slider mechanism portion 20 to move in a linear manner, the possibility of the operator making an operating error is suppressed to a low level, which is preferable in terms of suppressing failure in intraocular lens folding. More specifically, when the operator directly operates the slider mechanism portion 20 to move in a linear manner, depending on the operation method, this may result in an unintended usage pattern or variation in the usage pattern itself (it is difficult to perform the exact same operation every time), which may ultimately have a negative effect on the lens release performance, but according to the present embodiment, the operator does not need to directly operate the slider mechanism portion 20, thus making it possible to reduce operating errors, variation in usage pattern, and the like.

(e) The intraocular lens inserter 1 of the present embodiment is configured such that the rotatable actuator 50 and the slider mechanism portion 20 operate in conjunction with each other by utilizing engagement between the slider engagement groove 51 formed on the inner cylindrical surface of the rotatable actuator 50 and the slider engagement pieces 22 of the slider mechanism portion 20. In other words, operation of the rotatable actuator 50 and the slider mechanism portion 20 in conjunction with each other can be achieved with a very simple configuration. Therefore, the present embodiment is extremely preferable in view of realizing excellent operability (convenience) with a simplified configuration.

(f) In the intraocular lens inserter 1 of the present embodiment, the tacking pin 71 is provided in the cap member 3, and is arranged such that when the cap member 3 is in the attached state, the tacking pin 71 protrudes into the tube of the body portion 10 of the inserter main body 2. Therefore, when moving the intraocular lens inside the tube of the body portion 10, the tacking pin 71 can be used to ensure appropriate pre-folding of the intraocular lens. Furthermore, the tacking pin 71 only needs to be provided in association with the cap member 3, and does not need to be provided as a separate constituent element from the inserter main body 2 and the cap member 3, and thus there is no need to complicate the configuration of the intraocular lens inserter 1.

Furthermore, when the cap member 3 is removed from the body portion 10 of the inserter main body 2, the tacking pin 71 provided in the cap member 3 is configured to retract from the inside of the body portion 10 due to elastic deformation of the pin support portion 72. Therefore, the tacking pin 71 does not interfere with the operation for removal of the cap member 3, and the cap member 3 can be easily removed from the body portion 10 of the inserter main body 2, which also makes the intraocular lens inserter 1 extremely easy to operate (convenient) for the operator.

As described above, according to the present embodiment, the tacking pin 71 can be used to appropriately pre-fold the intraocular lens, while still achieving excellent convenience for the intraocular lens inserter 1 with a simplified configuration.

(g) In the intraocular lens inserter 1 according to the present embodiment, the intraocular lens push-out operation performed by the pushing mechanism portion 30 of the inserter main body 2 is restricted by the locking mechanism portion 40. Therefore, even if the cap member 3 does not cover the entirety of inserter main body 2 and the plunger end 32 and a part of the rod portion 31 that constitute the pushing mechanism portion 30 are exposed, the lens push-out operation performed by the pushing mechanism portion 30 is restricted, and the lens push-out operation is not performed by mistake.

Moreover, the locking mechanism portion 40 is configured to cancel the restriction on the push-out operation performed by the pushing mechanism portion 30, in conjunction with rotation of the rotatable actuator 50. Therefore, when using the intraocular lens inserter 1, the restriction imposed by the locking mechanism portion 40 is cancelled by rotating the rotatable actuator 50, and thus the restriction is cancelled in the one step for operating the rotatable actuator 50, and there is no need to perform a separate operation step to cancel the restriction.

As described above, according to the present embodiment, the restriction performed by the locking mechanism portion 40 and the canceling of such restriction are performed in conjunction with operation of the cap member 3, thus providing excellent operability (convenience) for the operator using the intraocular lens inserter 1.

### (4) Modifications, etc.

Although embodiments of the present invention have been described above, the above disclosure merely illustrates exemplary embodiments of the present invention. In other words, the technical scope of the present invention is not limited to the above-described exemplary embodiments, and various modifications are possible without departing from the spirit of the present invention.

In the above embodiment, the case where the inserter main body 2 includes the slider mechanism portion 20 and the locking mechanism portion 40 has been described as an example, but the present invention is not limited to this. In other words, as long as the inserter main body 2 includes at least the body portion 10 and the pushing mechanism portion 30, the intraocular lens can be released. In this case, if the cap member 3 is detachably attached to the inserter main body 2, it is possible to realize excellent convenience with a simplified configuration for the intraocular lens inserter 1.

Also, with regard to the pushing mechanism portion 30 that releases the intraocular lens, the case of a push type in which the intraocular lens is pushed out by performing a push-in operation on the plunger end 32 or the like has been described as an example in the above embodiment, but the present invention is not limited to this. For example, the pushing mechanism portion 30 is not limited to a push type, and may be a screw type, or may be compatible with both a push type and a screw type.

In the case where the slider mechanism portion 20 is provided, in the above embodiment, an example has been described in which the rotatable actuator 50 and the slider mechanism portion 20 are configured to operate in conjunction with each other, but the present invention is not limited to this. For example, the slider mechanism portion 20 may be configured such that movement of the intraocular lens is not performed in conjunction with operation of the rotatable actuator 50. However, if the rotatable actuator 50 and the slider mechanism portion 20 operate in conjunction with each other as in the above-described embodiment, operability (convenience) becomes excellent, which is preferable in terms of preventing failure in intraocular lens folding.

In the above embodiment, in the case where the rotatable actuator 50 and the slider mechanism portion 20 operate in conjunction with each other, the operation in conjunction with each other is achieved by engagement between the slider engagement pieces 22 and the slider engagement groove 51, but the present invention is not limited to this. For example, operation of the slider mechanism portion 20 and the rotatable actuator 50 in conjunction with each other may be achieved by other configurations, mechanisms, or the like.

Also, in the above embodiment, an example has been described in which the slider mechanism portion 20 functioning as one of the moving mechanism portions operates in conjunction with rotation of the rotatable actuator 50, but the present invention is not limited to this. For example, the pushing mechanism portion 30 functioning as another one of the moving mechanism portions may be configured to operate in conjunction with rotation of the rotatable actuator 50. In other words, in the present invention, it is only required that the moving mechanism portion for moving the intraocular lens in the body portion 10 is configured to operate in conjunction with rotation of the rotatable actuator 50.

In the case where the locking mechanism portion 40 is provided, in the above embodiment, the restriction performed by the locking mechanism portion 40 and the canceling of the restriction are performed in conjunction with the rotatable actuator 50, but the present invention is not limited to this. For example, the locking mechanism portion 40 may be configured such that restriction and canceling of restriction are not performed in conjunction with operation of the rotatable actuator 50. In this case, the locking mechanism portion 40 may be configured to perform restriction and canceling of restriction in conjunction with, for example, another operating means that is operable by the operator of the intraocular lens inserter 1. This means that the locking mechanism portion 40, which handles state switching of the operating member, does not necessarily require the presence of the rotatable actuator 50, and can also be configured to restriction the push-out operation performed by the pushing mechanism portion 30 even without the rotatable actuator 50.

Also, in the case where the locking mechanism portion 40 and the rotatable actuator 50 operate in conjunction with each other, in the above-described embodiment, an example has been described in which such operation in conjunction with each other is achieved by engagement between the lock engagement piece 42 and the lock engagement groove 52, but the present invention is not limited to this. For example, operation of the locking mechanism portion 40 and the rotatable actuator 50 in conjunction with each other may be achieved by other configurations, mechanisms, or the like.

Also, with regard to the locking mechanism portion 40, in the above embodiment, an example has been described in which the locking mechanism portion 40 has the locking member 41 that is rotatable around the cylindrical axis of the body portion 10, and when the locking member 41 rotates to the predetermined rotation position, the locking mechanism portion 40 switches the state from the restricted state to the operable state, but the present invention is not limited to this. For example, if the rod portion 31 of the pushing mechanism portion 30 is configured to be rotatable around the cylindrical axis of the body portion 10, the lock engagement piece 42 may be provided on the rod portion 31, and the state may be switched between the restricted state and the operable state according to the rotation position of the rod portion 31. In this case, the locking member 41 is not necessary, and the configuration of the locking mechanism portion 40 can be simplified accordingly. However, in the case of the configuration including the locking member 41, the push-out operation can be stably performed on the intraocular lens in a constant posture without rotation of the rod portion 31, which is very preferable for smoothly releasing the intraocular lens.

Furthermore, with regard to the locking mechanism portion 40, in the above embodiment, the locking mechanism portion 40 uses rotation of the locking member 41 to restrict and cancel restriction according to whether the rib 33 of the rod portion 31 and the guide groove 43 of the locking member 41 engage with or interfere with each other, but the present invention is not limited to this. For example, the restriction and canceling of restriction performed by the locking mechanism portion 40 may be achieved by other configurations, mechanisms, or the like. For example, as another mechanism, there is a so-called latch mechanism which restricts movement by abutting against a protrusion and cancels restriction by passing over the protrusion.

In the above-described embodiment, it is described that the constituent elements of the intraocular lens inserter 1 are formed from resin molded parts, but the present invention is not limited to this, and the constituent elements may be formed from metal parts made of stainless steel, titanium, or the like.

In the above embodiment, an example has been described in which the tacking pin 71 is retracted by elastic deformation of the pin support portion 72, but the present invention is not limited to this. For example, the deformation for retracting the tacking pin 71 may be plastic deformation.

### (5) Technical ideas in present specification

In view of the content of the embodiments described above, the present specification can be said to disclose technical ideas such as the following.

### [Supplementary Note 1]

An intraocular lens inserter including: an inserter main body having a hollow cylindrical portion capable of containing an intraocular lens, and configured to be able to release the intraocular lens contained in the hollow cylindrical portion to outside from a lens outlet;
moving mechanism portion that moves the intraocular lens within the hollow cylindrical portion, along a cylindrical axis direction of the hollow cylindrical portion;
a rotatable actuator attached to the inserter main body so as to be rotatable in a direction around a cylindrical axis of the hollow cylindrical portion; and
a cap member attached to the inserter main body so as to cover at least the lens outlet,
wherein the moving mechanism portion moves the intraocular lens in conjunction with a rotation of the rotatable actuator, and the cap member can be attached to and detached from the inserter main body while the rotatable actuator is at a predetermined rotation position.

### [Supplementary Note 2]

The intraocular lens inserter according to Supplementary Note 1,
wherein the moving mechanism portion has an engagement piece that protrudes outward of a cylinder of the hollow cylindrical portion, and
when the engagement piece engages with an engagement groove provided in the rotatable actuator, the rotatable actuator and the moving mechanism portion are in conjunction each other.

### [Supplementary Note 3]

The intraocular lens inserter according to Supplementary Note 2,
wherein the moving mechanism portion includes a slider mechanism portion that moves the intraocular lens within the hollow cylindrical portion.

### [Supplementary Note 4]

The intraocular lens inserter according to Supplementary Note 3,
wherein the engagement groove in the rotatable actuator extends spirally in a cylindrical axis direction of the hollow cylindrical portion.

### [Supplementary Note 5]

The intraocular lens inserter according to Supplementary Note 1,
wherein the cap member has:
a tacking pin arranged to protrude into a cylinder of the hollow cylindrical portion in the attached state; and
a pin support portion that supports the tacking pin,
wherein when the cap member is detached from the inserter main body, the tacking pin is retracted from inside of the cylinder of the hollow cylindrical portion due to deformation of the pin support portion.

### [Supplementary Note 6]

The intraocular lens inserter according to Supplementary Note 5,
wherein in addition to the tacking pin and the pin support portion, the cap member has a guide portion that causes deformation of the pin support portion in response to a relative movement between the cap member and the hollow cylindrical portion in the cylindrical axis direction of the hollow cylindrical portion.

### [Supplementary Note 7]

The intraocular lens inserter according to Supplementary Note 2,
wherein the moving mechanism portion includes a pushing mechanism portion that pushes out the intraocular lens from the lens outlet.

### [Supplementary Note 8]

The intraocular lens inserter according to Supplementary Note 7, including
a locking mechanism portion that restricts a pushing operation for the intraocular lens by the pushing mechanism portion.

### [Supplementary Note 9]

The intraocular lens inserter according to Supplementary Note 8,
wherein the locking mechanism portion is configured to cancel a restriction on the pushing operation, in conjunction with a rotation of the rotatable actuator.

### [Supplementary Note 10]

An intraocular lens inserter, which is an intraocular lens inserter for use with an intraocular lens, including an optical portion and an outer rim, the intraocular lens inserter including:
an inserter main body that defines an outer shape and a lens traveling direction, the inserter main body including:
a hollow cylindrical portion;
an intraocular lens storge area;
a nozzle;
a transition area between the intraocular lens storge area and the nozzle;
a plunger that is movable distally within the inserter main body in a lens
traveling direction relative to the inserter main body, having an end face configured to engage and push the intraocular lens during its distal movement;
a lens folding device that is movable distally in the lens traveling direction
relative to the inserter main body and the plunger, and configured to fold the intraocular lens during its distal movement; and
a rotatable actuator associated with an exterior of the inserter main body and operably connected to the lens folding device, so that the lens folding device moves distally in the lens traveling direction in response to the rotation of the rotatable actuator.

### [Supplementary Note 11]

The intraocular lens inserter according to Supplementary Note 10, including
a cap member detachably fixed to the inserter main body above the nozzle.

### [Supplementary Note 12]

The intraocular lens inserter according to Supplementary Note 11,
wherein the rotatable actuator is rotatable relative to the cap member.

### [Supplementary Note 13]

The intraocular lens inserter according to Supplementary Note 11 or 12,
wherein the cap member includes a first cap lock that is set in a locked state that prevents the cap member from being detached from the inserter main body, and an unlocked state that allows the cap member to be detached from the inserter main body, and
the lens folding device is configured to set the first cap lock to shift from the locked state to the unlocked state during a distal movement of the lens folding device.

### [Supplementary Note 14]

The intraocular lens inserter according to Supplementary Note 13,
wherein the inserter main body includes a latch opening, and
the first cap lock includes a latch that extends through the latch opening when the first cap lock is set in the locked state, and
the lens folding device includes a surface on which the latch moves relative to the latch opening when the lens folding device sets the first cap lock to shift from the locked state to the unlocked state.

### [Supplementary Note 15]

The intraocular lens inserter according to any one of Supplementary Notes 10 to 14,
wherein the second cap lock is further set in a locked state that prevents the cap member from being detached from the inserter main body, and an unlocked state that allows the cap member to be detached from the inserter main body, and
the second cap lock is set to shift from the locked state to the unlocked state by rotating the rotatable actuator.

### [Supplementary Note 16]

The intraocular lens inserter according to Supplementary Note 15,
wherein the second cap lock includes a pin and a slot in which the pin is placed.

### [Supplementary Note 17]

The intraocular lens inserter according to Supplementary Note 16,
wherein the cap member defines an inner surface, the pin is on the inner surface of the cap member, and the slot is positioned on a rotatable actuator and includes a circumferentially extending portion, a distally extending portion having an end portion connected to the circumferentially extending portion and an open end, and
when the rotatable actuator is fully rotated, the pin and the distally extending portion of the slot are aligned.

### [Supplementary Note 18]

The intraocular lens inserter according to any one of Supplementary Notes 10 to 17,
wherein the lens folding device includes a slider including an end face configured to engage with the outer rim of the intraocular lens, a hinge, and a lens holder connected to the hinge extending distally beyond the outer edge and onto an optical component of the intraocular lens.

### [Supplementary Note 19]

The intraocular lens inserter according to any one of Supplementary Notes 10 to 18,
wherein the hollow cylindrical portion defines a circumference and a central axis, and
the rotatable actuator extends completely around a circumference of a part of the hollow cylindrical portion and is rotatable around the central axis.

### [Supplementary Note 20]

The intraocular lens inserter according to Supplementary Note 19,
wherein the hollow cylindrical portion includes a cylindrical wall and at least one longitudinally extending slot extending through the cylindrical wall,
the rotatable actuator includes an inner surface with a helical thread, and
the lens folding device includes at least one post extending into the helical thread through at least one longitudinally extending slot.

### [Supplementary Note 21]

The intraocular lens inserter according to Supplementary Note 20,
wherein the hollow cylindrical portion includes first and second longitudinally extending slots extending through the cylindrical wall, and
the lens folding device includes first and second posts extending into the helical thread through the first and second longitudinally extending slots, respectively.

### [Supplementary Note 22]

The intraocular lens inserter according to any one of Supplementary Notes 11 to 21, further including
a detachable plug to prevent the rotatable actuator from rotating.

### [Supplementary Note 23]

The intraocular lens inserter according to Supplementary Note 22,
wherein the cap member includes a cylindrical wall and an opening passing through the cylindrical wall configured to receive the detachable plug,
the rotatable actuator includes a recess configured to receive a part of the detachable plug, and
the cap member, the rotatable actuator, and the plug each is constituted and arranged such that the cap member is fixed to the inserter main body, and the plug prevents a rotation of the rotatable actuator when the plug is positioned within the opening of the cap member and the recess of the rotatable actuator.

### [Supplementary Note 24]

The intraocular lens inserter according to any one of Supplementary Notes 10 to 23,
wherein the plunger lock is set in a locked state that prevents the plunger from moving distally and an unlocked state that allows the plunger to move distally, and
the plunger lock is set to shift from the locked state to the unlocked state by rotating the rotatable actuator.

### [Supplementary Note 25]

The intraocular lens inserter according to any one of Supplementary Notes 10 to 24,
which is further the intraocular lens stored in a lens storge area.

### [Supplementary Note 26]

An intraocular lens insertion method that is a method for inserting an intraocular lens into an eye using an intraocular lens inserter including an inserter main body that defines an exterior and a lens traveling direction, including a hollow cylindrical portion; an intraocular lens storge area; a nozzle; a transition area between the nozzle and the intraocular lens storge area; an intraocular lens in the lens storage area; and a plunger movable distally within the inserter main body relative to the inserter main body in a lens traveling direction and having an end face configured to engage and push the intraocular lens, wherein as the plunger moves distally, the rotatable actuator is associated with the exterior of the inserter main body, the intraocular lens insertion method including the following steps:
folding the intraocular lens without distally moving the plunger, by rotating the rotatable actuator; and after the intraocular lens has been folded,
pushing the intraocular lens through the nozzle and into the eye by moving the plunger distally.

### [Supplementary Note 27]

The intraocular lens insertion method according to Supplementary Note 26,
wherein the intraocular lens insertion device includes a lens folding device movable distally in a lens traveling direction with relative to the inserter main body and the plunger and configured to fold the intraocular lens during a distal movement of the lens folding device, and
the rotatable actuator is operably connected to the lens folding device such that the lens folding device moves distally in the lens traveling direction in response to the rotation of the rotatable actuator.

### [Supplementary Note 28]

The intraocular lens insertion method according to Supplementary Note 26 or 27,
wherein the intraocular lens insertion device includes a cap member on the inserter main body that covers the nozzle, and a cap lock that is set in a locked state so that the cap member is not detached from the inserter main body, and the intraocular lens insertion method further includes:
setting the cap lock to shift to an unlocked state in which the cap lock can be detached from the inserter main body during the folding step; and
detaching the cap member before the pushing step.

### [Supplementary Note 29]

The intraocular lens insertion method according to any one of Supplementary Notes 26 to 28,
wherein the intraocular lens insertion device includes a plunger lock set in a locked state that prevents a distal movement of the plunger,
the method further including:
   setting the plunger lock to shift to an unlocked state that allows the plunger to move distally during the folding step.

### [Supplementary Note 30]

The intraocular lens insertion method according to any one of Supplementary Notes 26 to 29,
wherein the hollow cylindrical portion defines a circumference and a central axis, and
the rotatable actuator extends completely around a part of the hollow cylindrical portion and is rotatable around the central axis,
the step of rotating the rotatable actuator further including:
   rotating the rotatable actuator around the central axis.

### Reference Signs List

1 intraocular lens inserter, 2 inserter main body, 3 cap member, 4 stopper member, 10 body portion, 11 base portion, 12 nozzle portion, 13 flange portion, 14 lens housing portion, 15 slit hole, 16 guide hole, 18 lens outlet, 19 transition portion, 20 slider mechanism portion, 21 lens support member, 22 slider engagement piece, 30 pushing mechanism portion, 31 rod portion, 32 plunger end, 40 locking mechanism portion, 41 locking member, 42 lock engagement piece, 50 rotatable actuator, 51 slider engagement groove, 52 lock engagement groove, 53 fitting portion, 54 cap engagement groove, 55 rotation directional groove, 56 insertion/removal directional groove, 70 tacking assist portion, 71 tacking pin, 72 pin support portion, 73 guide portion

## Claims

1. An intraocular lens inserter comprising:
an inserter main body having a hollow cylindrical portion capable of containing an intraocular lens, and configured to be able to release the intraocular lens contained in the hollow cylindrical portion to outside from a lens outlet;
moving mechanism portion that moves the intraocular lens within the hollow cylindrical portion, along a cylindrical axis direction of the hollow cylindrical portion;
a rotatable actuator attached to the inserter main body so as to be rotatable in a direction around a cylindrical axis of the hollow cylindrical portion;
and
a cap member attached to the inserter main body so as to cover at least the lens outlet,
wherein the moving mechanism portion moves the intraocular lens in conjunction with a rotation of the rotatable actuator, and the cap member can be attached to and detached from the inserter main body while the rotatable actuator is at a predetermined rotation position.

2. The intraocular lens inserter according to claim 1,
wherein the moving mechanism portion has an engagement piece that protrudes outward of a cylinder of the hollow cylindrical portion, and
when the engagement piece engages with an engagement groove provided in the rotatable actuator, the rotatable actuator and the moving mechanism portion are in conjunction each other.

3. The intraocular lens inserter according to claim 2, wherein the moving mechanism portion includes a slider mechanism portion that moves the intraocular lens within the hollow cylindrical portion.

4. The intraocular lens inserter according to claim 3, wherein the engagement groove in the rotatable actuator extends spirally in a cylindrical axis direction of the hollow cylindrical portion.

5. The intraocular lens inserter according to claim 1, wherein the cap member has:
a tacking pin arranged to protrude into a cylinder of the hollow cylindrical portion in the attached state; and
a pin support portion that supports the tacking pin,
wherein when the cap member is detached from the inserter main body, the tacking pin is retracted from inside of the cylinder of the hollow cylindrical portion due to deformation of the pin support portion.

6. The intraocular lens inserter according to claim 5, wherein in addition to the tacking pin and the pin support portion, the cap member has a guide portion that causes deformation of the pin support portion in response to a relative movement between the cap member and the hollow cylindrical portion in the cylindrical axis direction of the hollow cylindrical portion.

7. The intraocular lens inserter according to claim 2, wherein the moving mechanism portion includes a pushing mechanism portion that pushes out the intraocular lens from the lens outlet.

8. The intraocular lens inserter according to claim 7, comprising a locking mechanism portion that restricts a pushing operation for the intraocular lens by the pushing mechanism portion.

9. The intraocular lens inserter according to claim 8, wherein the locking mechanism portion is configured to cancel a restriction on the pushing operation, in conjunction with a rotation of the rotatable actuator.

10. An intraocular lens inserter, which is an intraocular lens inserter for use with an intraocular lens, including an optical portion and an outer rim, the intraocular lens inserter including:
an inserter main body that defines an outer shape and a lens traveling direction, the inserter main body including:
a hollow cylindrical portion;
an intraocular lens storge area;
a nozzle;
a transition area between the intraocular lens storge area and the nozzle;
a plunger that is movable distally within the inserter main body in a lens traveling direction relative to the inserter main body, having an end face configured to engage and push the intraocular lens during its distal movement;
a lens folding device that is movable distally in the lens traveling direction relative to the inserter main body and the plunger, and configured to fold the intraocular lens during its distal movement; and
a rotatable actuator associated with an exterior of the inserter main body and operably connected to the lens folding device, so that the lens folding device moves distally in the lens traveling direction in response to the rotation of the rotatable actuator.

11. The intraocular lens inserter according to claim 10, including a cap member detachably fixed to the inserter main body above the nozzle.

12. The intraocular lens inserter according to claim 11, wherein the rotatable actuator is rotatable relative to the cap member.

13. The intraocular lens inserter according to claim 11 or 12,
wherein the cap member includes a first cap lock that is set in a locked state that prevents the cap member from being detached from the inserter main body, and an unlocked state that allows the cap member to be detached from the inserter main body, and
the lens folding device is configured to set the first cap lock to shift from the locked state to the unlocked state during a distal movement of the lens folding device.

14. The intraocular lens inserter according to claim 13,
wherein the inserter main body includes a latch opening, and
the first cap lock includes a latch that extends through the latch opening when the first cap lock is set in the locked state, and
the lens folding device includes a surface on which the latch moves relative to the latch opening when the lens folding device sets the first cap lock to shift from the locked state to the unlocked state.

15. The intraocular lens inserter according to any one of claims 10 to 14, wherein the second cap lock is further set in a locked state that prevents the cap member from being detached from the inserter main body, and an unlocked state that allows the cap member to be detached from the inserter main body, and
the second cap lock is set to shift from the locked state to the unlocked state by rotating the rotatable actuator.

16. The intraocular lens inserter according to claim 15, wherein the second cap lock includes a pin and a slot in which the pin is placed.

17. The intraocular lens inserter according to claim 16,
wherein the cap member defines an inner surface, the pin is on the inner surface of the cap member, and the slot is positioned on a rotatable actuator and includes a circumferentially extending portion, a distally extending portion having an end portion connected to the circumferentially extending portion and an open end, and
when the rotatable actuator is fully rotated, the pin and the distally extending portion of the slot are aligned.

18. The intraocular lens inserter according to any one of claims 10 to 17, wherein the lens folding device includes a slider including an end face configured to engage with the outer rim of the intraocular lens, a hinge, and a lens holder connected to the hinge extending distally beyond the outer edge and onto an optical component of the intraocular lens.

19. The intraocular lens inserter according to any one of claims 10 to 18,
wherein the hollow cylindrical portion defines a circumference and a central axis, and
the rotatable actuator extends completely around a circumference of a part of the hollow cylindrical portion and is rotatable around the central axis.

20. The intraocular lens inserter according to claim 19,
wherein the hollow cylindrical portion includes a cylindrical wall and at least one longitudinally extending slot extending through the cylindrical wall,
the rotatable actuator includes an inner surface with a helical thread, and
the lens folding device includes at least one post extending into the helical thread through at least one longitudinally extending slot.

21. The intraocular lens inserter according to claim 20,
wherein the hollow cylindrical portion includes first and second longitudinally extending slots extending through the cylindrical wall, and
the lens folding device includes first and second posts extending into the helical thread through the first and second longitudinally extending slots, respectively.

22. The intraocular lens inserter according to any one of claims 11 to 21, further comprising a detachable plug to prevent the rotatable actuator from rotating.

23. The intraocular lens inserter according to claim 22,
wherein the cap member includes a cylindrical wall and an opening passing through the cylindrical wall configured to receive the detachable plug,
the rotatable actuator includes a recess configured to receive a part of the detachable plug, and
the cap member, the rotatable actuator, and the plug each is constituted and arranged such that the cap member is fixed to the inserter main body, and the plug prevents a rotation of the rotatable actuator when the plug is positioned within the opening of the cap member and the recess of the rotatable actuator.

24. The intraocular lens inserter according to any one of claims 10 to 23,
wherein the plunger lock is set in a locked state that prevents the plunger from moving distally and an unlocked state that allows the plunger to move distally, and
the plunger lock is set to shift from the locked state to the unlocked state by rotating the rotatable actuator.

25. The intraocular lens according to any one of claims 10 to 24, which is further the intraocular lens stored in a lens storge area.

26. An intraocular lens insertion method that is a method for inserting an intraocular lens into an eye using an intraocular lens inserter including an inserter main body that defines an exterior and a lens traveling direction, comprising a hollow cylindrical portion; an intraocular lens storge area; a nozzle; a transition area between the nozzle and the intraocular lens storge area; an intraocular lens in the lens storage area; and a plunger movable distally within the inserter main body relative to the inserter main body in a lens traveling direction and having an end face configured to engage and push the intraocular lens, wherein as the plunger moves distally, the rotatable actuator is associated with the exterior of the inserter main body,
the intraocular lens insertion method comprising:
folding the intraocular lens without distally moving the plunger, by rotating the rotatable actuator; and after the intraocular lens has been folded,
pushing the intraocular lens through the nozzle and into the eye by moving the plunger distally.

27. The intraocular lens insertion method according to claim 26,
wherein the intraocular lens insertion device includes a lens folding device movable distally in a lens traveling direction with relative to the inserter main body and the plunger and configured to fold the intraocular lens during a distal movement of the lens folding device, and
the rotatable actuator is operably connected to the lens folding device such that the lens folding device moves distally in the lens traveling direction in response to the rotation of the rotatable actuator.

28. The intraocular lens insertion method according to claim 26 or 27,
wherein the intraocular lens insertion device includes a cap member on the inserter main body that covers the nozzle, and a cap lock that is set in a locked state so that the cap member is not detached from the inserter main body,
the method further including:
setting the cap lock to shift to an unlocked state in which the cap lock can be detached from the inserter main body during the folding step; and
detaching the cap member before the pushing step.

29. The intraocular lens insertion method according to any one of claims 26 to 28,
wherein the intraocular lens insertion device includes a plunger lock set in a locked state that prevents a distal movement of the plunger,
the method further comprising:
setting the plunger lock to shift to an unlocked state that allows the plunger to move distally during the folding step.

30. The intraocular lens insertion method according to any one of claims 26 to 29,
wherein the hollow cylindrical portion defines a circumference and a central axis, and
the rotatable actuator extends completely around a part of the hollow cylindrical portion and is rotatable around the central axis,
the step of rotating the rotatable actuator further including:
rotating the rotatable actuator around the central axis.
